Europäisches Patentamt

**European Patent Office**

Office européen des brevets

(19)

(11) Veröffentlichungsnummer. **0 058 940**
**B1**

(12) **EUROPÄISCHE PATENTSCHRIFT**

(45) Veröffentlichungstag der Patentschrift:
27.06.84

(21) Anmeldenummer: 82101208.5

(22) Anmeldetag: 18.02.82

(51) Int. Cl.³: **C 07 C 29/136,** C 07 C 29/17,
C 07 C 31/20

(54) Verfahren zur Herstellung von 1,4-Butandiol.

(30) Priorität: **24.02.81 DE 3106819**

(43) Veröffentlichungstag der Anmeldung:
**01.09.82 Patentblatt 82/35**

(45) Bekanntmachung des Hinweises auf die Patenterteilung:
**27.06.84 Patentblatt 84/26**

(84) Benannte Vertragsstaaten:
**BE DE FR GB IT NL**

(56) Entgegenhaltungen:
**BE - A - 661 258**
**DE - A - 2 715 667**
**FR - A - 2 286 808**
**FR - A - 2 293 410**
**FR - A - 2 439 176**
**US - A - 2 040 944**
**US - A - 4 032 458**

**Römpps Chemie-Lexikon, 8. Auflage, Seite 112 (1979)**
**Franck'sche Verlagsbuchhandlung Stuttgart (DE)**

(73) Patentinhaber: **BASF Aktiengesellschaft,
Carl-Bosch-Strasse 38, D-6700 Ludwigshafen (DE)**

(72) Erfinder: **Weitz, Hans-Martin, Dr., Auf dem Koeppel 40,
D-6702 Bad Duerkheim (DE)**
Erfinder: **Schnabel, Rolf, Dr., Tilsiter Strasse 9,
D-6707 Schifferstadt (DE)**
Erfinder: **Platz, Rolf, Dr., Hansastrasse 5,
D-6800 Mannheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Diese Erfindung betrifft ein Verfahren zur Herstellung von 1,4-Butandiol, ausgehend von Maleinsäureanhydrid durch katalytische Hydrierung in Gegenwart von 1,4-Butandiol als Lösungsmittel.

1,4-Butandiol und Tetrahydrofuran sind bekanntlich wertvolle Zwischenprodukte, die z. B. für die Herstellung von Polyurethanen, Polybutylenterephthalat oder Polytetrahydrofuran verwendet werden.

Es sind bereits mehrere Wege für die großtechnische Gewinnung von 1,4-Butandiol bzw. Tetrahydrofuran beschrieben worden. Der größte Teil des in der Technik hergestellten 1,4-Butandiols bzw. Tetrahydrofurans wird über Butindiol gewonnen, welches nach der Reppe-Synthese aus Acetylen und Formaldehyd hergestellt wird. Der in letzter Zeit stark ansteigende Preis des Acetylens verschlechtert jedoch die Wirtschaftlichkeit dieses Syntheseweges. Nach einem anderen, in den letzten Jahren entwickelten Verfahren werden Butandiene acetoxyliert und die hierbei als Zwischenprodukte anfallenden Butendioldiacetate durch katalytische Hydrierung und anschließende Hydrolyse in 1,4-Butandiol übergeführt. Tetrahydrofuran kann hierbei in bekannter Weise aus dem Butandiol oder aus dem intermediär anfallenden Butandioldiacetat durch Cyclisierung gewonnen werden.

Es ist auch schon vorgeschlagen worden, 1,4-Butandiol durch katalytische Hydrierung von Maleinsäureanhydrid herzustellen. Nach dem in der DE-OS 2 543 673 beschriebenen Verfahren hydriert man ein Gemisch, das durch Behandlung von Maleinsäureanhydrid mit aliphatischen Alkoholen bei höheren Temperaturen erhalten wurde. Bei der Hydrierung entsteht aus dem in dem Gemisch vorliegenden 1-Butendicarbonsäurealkylat durch Hydrogenolyse das 1,4-Butandiol, während man den verwendeten Alkohol zurückgewinnt. Aus der DE-OS 2 845 905 ist bekannt, daß man 1, 4-Butandiol auch herstellen kann, wenn man eine Lösung von Maleinsäureanhydrid in einem aliphatischen Alkohol in einer Stufe bei Temperaturen von 180 bis 300°C und Drücken von 250 bis 350 bar an Kupferchromit-Katalysatoren hydriert. Als Alkohole verwendet man für diese Verfahren einwertige aliphatische Alkohole mit 1 bis 6 C-Atomen. Ein ähnliches Verfahren, bei dem man zu dem Gemisch aus Maleinsäureanhydrid Alkylorthotitanate als Veresterungskatalysatoren gibt, wird in der FR-A-2 293 410 beschrieben. Bei einem in der US-A-4 032 458 beschriebenen Verfahren wird die Lösung von Maleinsäureanhydrid in einem einwertigen Alkohol mit 2 bis 10 C-Atomen in mehreren Stufen hydriert. Bei diesem Verfahren ist es erforderlich, die als Lösungsmittel verwendeten Alkohole vom Butandiol abzutrennen.

Da die Isolierung von Maleinsäureanhydrid aus den Reaktionsgasen, die bei der Luftoxidation von Kohlenwasserstoffen, wie Benzol, Butenen oder Butan entstehen, erhebliche technische Schwierigkeiten bereitet und bei der Hydrierung von Maleinsäureanhydrid Korrosionsprobleme durch die Bildung von Wasser und damit von freier Maleinsäure und Bernsteinsäure auftreten, ist die Wirtschaftlichkeit dieser Verfahren schlecht.

Es wurde nun gefunden, daß man 1,4-Butandiol erheblich vorteilhafter herstellen kann, wenn man Maleinsäureanhydrid enthaltende gasförmige Reaktionsgemische, wie man sie durch katalytische Luftoxidation von Benzol, Butenen oder Butan erhält, mit mehr als der 2molaren Menge, bezogen auf 1 Mol Maleinsäureanhydrid, an 1,4-Butandiol wäscht, die dabei erhaltene Lösung des Maleinsäurehalbesters in 1,4-Butandiol unter gleichzeitiger Entfernung des Wassers auf Temperaturen von 120 bis 150°C erhitzt, und das Gemisch aus Diester und 1,4-Butandiol auf an sich bekannte Weise bei Temperaturen von 180 bis 220°C und Drucken von 180 bis 400 bar hydriert.

Maleinsäureanhydrid enthaltende gasförmige Reaktionsgemische, wie man sie beim erfindungsgemäßen Verfahren benötigt, werden nach an sich bekannten Verfahren der katalytischen Luftoxidation von Benzol, Butenen oder Butan erhalten. Bei diesen bekannten Oxidationsverfahren strömt aus dem Reaktor ein gasförmiges Reaktionsgemisch mit einer Temperatur von 250 bis 600°C, das je $Nm^3$ bis 40 g Maleinsäureanhydrid enthält. Außerdem enthalten die gasförmigen Reaktionsgemische neben nicht umgesetztem Kohlenwasserstoff Wasser, Kohlenmonoxid und Kohlendioxid.

Zur Absorption des Maleinsäureanhydrids leitet man das Maleinsäureanhydrid enthaltende Reaktionsgas z. B. in eine Kolonne, in die man gleichzeitig das 1,4-Butandiol einleitet. Der Alkohol wird in mehr als der 2molaren Menge, bezogen auf 1 Mol Maleinsäureanhydrid, angewendet. Vorzugsweise verwendet man eine 5 bis 25fache molare Menge, bezogen auf das Maleinsäureanhydrid. Man betreibt die Waschkolonne zweckmäßig mit einer Temperatur von 120 bis 160°C und vorteilhaft unterhalb des Siedepunktes des Butandiols. Die Absorption wird im Gleichstrom oder im Gegenstrom vorgenommen. Dabei wird das Maleinsäureanhydrid durch den Alkohol aus dem gasförmigen Reaktionsgemisch herausgewaschen, wobei sich überwiegend die Halbester der Maleinsäure und Fumarsäure bilden. Anteile des Alkohols, die in die Gasphase gelangen, werden aus dem Abgas der Waschkolonne zurückgewonnen.

Man erhitzt die Lösung des Maleinsäurehalbesters in dem Alkohol, die man in der Waschkolonne gewinnt, auf Temperaturen von 120 bis 150°C, wobei der entsprechende Diester gebildet wird. Das Reaktionswasser entfernt man durch Destillation.

Das Gemisch aus Diester und 1,4-Butandiol wird schließlich der katalytischen Hydrierung unterworfen. Man hydriert auf an sich bekannte Weise bei Temperaturen von 180 bis 220°C und Drucken von 180 bis 400 bar, wobei man an sich übliche Hydrierungskatalysatoren, wie die im Report 96A des Stanfort Research Institut (Nov. 1977) Seiten 122 bis 126 beschriebenen Katalysatoren verwendet. Besonders vorteilhaft sind z. B. Trägerkatalysatoren, die 20 bis 40 Gew.-% Kupfer enthalten, wobei das Kupfer noch geringe Mengen anderer Metalle, wie Chrom enthalten kann.

Beispiel

In einer Laborglockenbodenkolonne aus Glas (8 praktische Böden) wird aus einem gasförmigen Reaktionsgemisch mit einer Temperatur von 150°C (500 l/h), das durch katalytische Luftoxidation eines $C_4$-Schnittes erhalten wurde, und das je 100 l 3 g Maleinsäureanhydrid und 3,5 g Wasser enthält, mit 250 ml/h 1,4-Butandiol der Temperatur 150°C das Maleinsäureanhydrid ausgewaschen.

Die erhaltene Lösung erhitzt man zur Nachveresterung auf 140°C. Dabei gebildetes Wasser wird zusammen mit dem ebenfalls entstandenen Tetrahydrofuran abdestilliert.

Die Lösung von Maleinsäure-di-(4-hydroxybutyl)-ester in 1,4-Butandiol wird an einem CuO/$Cr_2O_3$-Kieselsäure-Katalysator (25 Gew.-% CuO, 1 Gew.-% $Cr_2O_3$) bei 195°C und einem Druck von 200 bar mit Wasserstoff behandelt. Man erhält 1,4-Butandiol mit einem Gehalt an Tetrahydrofuran von 5 Gew.-%, ber. auf Butandiol, das aus Maleinsäureanhydrid gebildet wurde. Die Reinheit des destillativ gereinigten 1,4-Butandiols liegt bei 99,5% (GC-Analyse).

Für eine Wiederverwendung in der Absorption muß das bei der Hydrierung anfallende rohe Butandiol nicht oder nur teilweise destillativ aufgearbeitet werden.

**Patentansprüche**

1. Verfahren zur Herstellung von 1,4-Butandiol, dadurch gekennzeichnet, daß man Maleinsäureanhydrid enthaltende gasförmige Reaktionsgemische, wie man sie durch katalytische Luftoxidation von Benzol, Butenen oder Butan erhält, mit mehr als der 2molaren Menge, bezogen auf 1 Mol Maleinsäureanhydrid, an 1,4-Butandiol wäscht, die dabei erhaltene Lösung des Maleinsäurehalbesters in 1, 4-Butandiol unter gleichzeitiger Entfernung des Wassers auf Temperaturen von 120 bis 150°C erhitzt, und das Gemisch aus gebildetem Diester und 1,4-Butandiol auf an sich bekannte Weise bei Temperaturen von 180 bis 220°C und Drücken von 180 bis 400 bar hydriert.

2. Verfahren nach Anspruch 1, dadurch gekennzeichnet, daß man die Wäsche der das Maleinsäureanhydrid enthaltenden gasförmigen Reaktionsgemische mit dem 1,4-Butandiol in einer Kolonne bei Temperaturen von 120 bis 160°C vornimmt.

**Claims**

1. A process for the preparation of butane-1,4-diol, wherein a gaseous reaction mixture, which contains maleic anhydride and is obtained in the catalytic oxidation of benzene, butenes or butane by air, is washed with more than twice the molar amount, based on 1 mole of maleic anhydride, of butane-1,4-diol, the resulting solution of the half-ester of maleic acid in butane-1,4-diol is heated to 120 to 150°C while simultaneously removing the water, and the mixture of the diester formed and butane-1,4-diol is hydrogenated in a conventional manner at from 180 to 220°C and under a pressure of from 180 to 400 bar.

2. A process as claimed in claim 1, wherein the gaseous reaction mixture containing the maleic anhydride is washed with butane-1,4-diol in a column at from 120 to 160°C.

**Revendications**

1. Procédé de préparation du butane diol-1,4, caractérisé en ce que l'on soumet des mélanges réactionnels, obtenus par une oxydation catalysée du benzène, de butènes ou de butane par l'air et contenant de l'anhydride maléique, à l'état gazeux à un lavage au butane diol-1,4 en une proportion plus que 2 molaire par mole d'anhydride maléique, on chauffe ensuite la solution du hémi-ester de l'acide maléique dans le butane diol-1,4 obtenue à des températures de 120 à 150°C avec élimination concomitante de l'eau, puis on soumet le mélange du di-ester formé et de butane diol-1,4 d'une manière connue en soi à une hydrogénation à des températures de 180 à 220°C sous des pressions de 180 à 400 bars.

2. Procédé suivant la revendication 1, caractérisé en ce que le lavage du mélange réactionnel gazeux, contenant de l'anhydride maléique, au butane diol-1,4 est réalisé dans une colonne à des températures de 120 à 160°C.